# FASCICULE DE BREVET EUROPEEN

(11) **EP 0 750 041 B1**
(45) Date de publication et mention de la délivrance du brevet: **10.12.2008**
(21) Numéro de dépôt: 96108720.2
(22) Date de dépôt: 15.01.1988
(51) Int. Cl.: C12N 15/48, C07K 14/16, C12Q 1/68

(54) **Séquences de nucléotides de HIV-2 et SIV-1**
HIV-2 und SIV-1 Nukleotidsequenzen
HIV-2 and SIV-1 nucleotide sequences

(30) Priorité: 16.01.1987 US 3764; 11.02.1987 FR 8701739; 15.04.1987 FR 8705398
(43) Date de publication de la demande: 27.12.1996
(62) Demande divisionnaire de: 88400084.5
(73) Titulaire: INSTITUT PASTEUR, 75015 Paris (FR); CENTRE NATIONAL DE LA RECHERCHE SCIENTIFIQUE, 75007 Paris (FR)
(72) Inventeur: Alizon, Marc, 75005 Paris (FR); Montagnier, Luc, 92350 Le Plessis Robinson (FR); Guetard, Denise, 75015 Paris (FR); Clavel, Françoise, Rockville, MD 20852 (US); Sonigo, Pierre, 75015 Paris (FR); Guyader, Mireille, 75017 Paris (FR); Tiollais, Pierre, 75013 Paris (FR); Chakrabarti, Lisa, 750 Paris (FR); Desrosiers, Ronald, Hudson, Massachusetts 01749 (US)
(74) Mandataire: Desaix, Anne

(56) Documents cités:
- EP-A- 0 239 425
- EP-A- 0 269 520
- NATURE, vol. 324, Décembre 1986, pages 691-695, XP000605290 CLAVEL, F. ET AL.: "Molecular cloning and polymorphism of the human immune deficiency virus type 2"
- VACCINES 87, Janvier 1987, pages 179-184, XP000604420 CLAVEL, F. ET AL.: "HIV-2, the causative agent of AIDS in West Africa"
- NATURE, vol. 326, 16 Avril 1987, pages 662-669, XP002018635 GUYADER, M. ET AL.: "Genome organization and transactivation of the human immunodeficiency virus type 2"

## Description

La présente invention décrit des peptides ayant des propriétés immunologiques, le cas échéant immunogènes, en commun avec des antigènes susceptibles d'être obtenus sous une forme purifiée, à partir de virus capables de provoquer des lymphadénopathies susceptibles d'être relayées ensuite par le symdrôme d'immunodéficience acquise (SIDA) chez l'homme.

La demande dècrit en particulier des peptides antigéniques susceptibles d'être reconnus par des anticorps induits chez l'homme par des virus désignés par l'abréviation HIV, selon la nomenclature définie dans NATURE. Elle concerne également des peptides ayant des propriétés immunogènes ou susceptibles d'être rendus immunogènes in vivo, cette immunogénicité étant susceptible de se manifester par l'induction in vivo d'anticorps reconnaissant des antigènes caractéristiques des virus HIV-2 et même, au moins en ce qui concerne certains de ces peptides, des antigènes issus de HIV-1.

La demande dècrit en outre des applications de ces peptides à la fabrication de compositions pour le diagnostic in vitro chez l'homme de potentialité de certaines formes du SIDA et, en ce qui concerne certains d'entre eux, à la production de compositions immunogènes et de compositions vaccinantes contre les rétrovirus HIV.

De même la demande décrit les applications aux mêmes fins des anticorps susceptibles d'être induits in vivo par les peptides immunogènes ou rendus immunogènes et, pour certains de ces anticorps, leurs applications à la production de principes actifs de médicaments contre ces SIDAS humains.

La demande décrit également la mise en oeuvre de certains de ces peptides dans des procédés pour le diagnostic in vitro chez l'homme de certaines formes du SIDA, ainsi que leur application à la constitution de trousses ou "kits" de diagnostic.

Un premier rétrovirus dénommé LAV-1 ou HIV-1 a été isolé et décrit dans la demande de brevet GB.83/24.800 et une demande EP.84/401.834 du 14/09/84. Ce virus a également été décrit par F.Barre Sinoussi et al. dans Science, 220 n° 45-99, 20 pages 868-871.

Des variants de ce virus HIV-1 désignés par LAV ELI et LAV MAL, ont également été isolés, caractérisés et décrits dans la demande de brevet EP.84/- 401.834.

Les virus HIV-1 et leurs variants possèdent les propriétés suivantes :
- ils ont pour cibles préférencielles les cellules Leu3 (ou lymphocytes T4) humaines et leurs cellules dérivées "immortalisées".
- ils ont une activité transcriptase inverse nécessitant la présence d'ions Mg²⁺ et présentent une forte activité pour le poly(adenylate-oligo-deoxythymidylase) poly(A)-oligo(dT)12-18)
- ils ont une densité de 1,16 à 1,17 sur gradient de sucrose,
- ils ont un diamètre moyen de 139 nanomètres et un noyau de diamètre moyen de 41 nanomètres,
- les lysats de ces virus contiennent une protéine p25 (protéine du noyau) qui ne croise pas immunologiquement avec la protéine p24 de HTLV-1,
- ils contiennent une protéine p42 appartenant à leur enveloppe,
- ils contiennent également une glycoprotéine d'enveloppe gp110 d'un poids moléculaire de 110.000.

L'isolement et la caractérisation de rétrovirus appartenant à une classe distincte et n'ayant qu'une parenté immunologique réduite avec les précédents, ont été décrits dans la demande de brevet européen n° 87/400.151.4. Ces rétrovirus qui ont été regroupés sous la désignation HIV-2, ont été isolés chez plusieurs malades africains présentant des symptômes d'une lymphadénopathie ou d'un SIDA.

Les rétrovirus du type HIV-2 comme les rétrovirus du type HIV-1, se caractérisent par un tropisme pour les lymphocytes T4 humains et par un effet cytopathogène à l'égard de ces lymphocytes, lorsqu'ils s'y multiplient, pour alors causer soit des poly-adénopathies généralisées et persistantes, soit un SIDA.

Plus généralement les rétrovirus purifiés par HIV-2 possèdent en général les propriétés suivantes :
- la cible préférentielle des rétrovirus HIV-2 est constituée par les cellules Leu3 (ou lymphocytes T4) humaines et pour des cellules "immortalisées" dérivées de ces lymphocytes T4 ;
- ils sont cytotoxiques pour les lymphocytes T4 humains
- ils ont une activité de transcriptase inverse nécessitant la présence d'ions Mg²⁺ et présentant une forte activité pour le poly(adénylate-oligodéoxythylmidylase) (poly(A)-oligo(dT) 12-18) ;
- ils ont une densité de 1,16 dans un gradient de sucrose ;
- ils ont un diamètre moyen de 140 nanomètres et un noyau ayant un diamètre moyen de 41 nanomètres ;
- ils peuvent être cultivés dans des lignées permanentes du type HUT ou exprimant la protéine T4 ;
- ils ne sont pas infectieux pour les lymphocytes T8 ;
- les lysats de ces virus contiennent une protéine p26 qui ne croise pas immunologiquement avec la protéine p24 du virus HTLV-I ou du virus HTLV-II ;
- ces lysats contiennent en outre une protéine p16 qui n'est pas reconnue immunologiquement par la protéine p19 de HTLV-I ou de HTLV-II dans des essais de radioimmuno-précipitation ;
- ils contiennent en outre une glycoprotéine d'enveloppe ayant un poids moléculaire de l'ordre de 130.000-140.000 qui ne croise pas immunologiquement avec la gp110 des HIV-1, mais qui en revanche croise immunologiquement avec la glycoprotéine d'enveloppe gp140 de STLV-III (virus isolé chez le singe) ;
- ces lysats contiennent encore des antigènes marquables par la ³⁵S-cystéine, dont les poids moléculaires s'étagent entre 32.000 et 42.000-45.000 : ils comprennent notamment un antigène ayant un poids moléculaire de l'ordre de 36.000 et un antigène ayant un poids moléculaire de l'ordre de 42.000, l'un de ces antigènes (p36 et p42) constituant vraisemblablement une glycoprotéine transmembranaire du virus HIV-2 ;
- l'ARN génomique des HIV-2 n'hybride pas avec l'ARN génomique de HIV-1 dans des conditions stringentes ;
- dans des conditions non stringentes, l'ARN génomique de HIV-2 n'hybride, ni avec le gène env et le LTR qui le jouxte, de HIV-1, ni avec des séquences de la région pol du génome de HIV-1 ;
- dans des conditions non stringentes, il hybride faiblement avec des séquences de nucléotides de la région de HIV-1.

Un autre rétrovirus dénommé SIV-1, cette dénomination remplaçant la dénomination antérieurement connue STLV III, a été isolé chez le singe macaque rhésus. (M.D.Daniel et al. Science 228, 1201 (1985) N.L.Letwin et al, Science 230, 71 (1985) sous l'appellation "STLV-IIImac").

Un autre rétrovirus, désigné "STLV-III_{AGM}", (ou SIV_{AGM}) a été isolé chez des singes verts sauvages. Mais, contrairement au virus présent chez le singe macaque rhésus, la présence de "STLV-III_{AGM}" ne semble pas induire une maladie du type SIDA chez le singe vert d'Afrique. Une souche du rétrovirus SIV-1mac a été déposée à la CNCM le 7 Février 1986 sous le n° I-521. Des études ont montré que le rétrovirus SIV-1 comporte certaines protéines possédant une certaine parenté immunologique avec des protéines ou glycoprotéines structurales susceptibles d'être obtenues dans des conditions analogues, à partir de HIV-2. Ce rétrovirus SIV-1, dont on a constaté le caractère infectieux chez les singes, avait été désigné par STLVIII par les chercheurs qui l'ont isolé (références bibliographiques précitées).

Pour la commodité du langage, ces virus ne seront plus désignés dans ce qui suit que par l'expression SIV (l'expression SIV est l'abréviation anglaise de "Simian Immunodeficiency Virus" (virus d'immunodéficience du singe)) éventuellement suivi d'une abréviation désignant l'espèce de singe dont ils sont issus, par exemple, MAC (ou mac) pour le macaque ou AGM pour le singe vert d'Afrique (abréviation de "African Green Monkey").

En mettant en oeuvre les mêmes techniques que celles rappelées plus haut, il a été constaté que l'on pouvait également obtenir à partir de SIV-1mac :
- une protéine principale du noyau p27, ayant un poids moléculaire de l'ordre de 27 kilodaltons,
- une glycoprotéine majeure d'enveloppe, gp140,
- une protéine vraisemblablement transmembranaire p32, qui n'est guère observée en RIPA lorsque le virus a au préalable été marqué par la ³⁵S-cystéine, mais qui peut être observée dans les essais d'immunoempreintes (Western blots), sous forme de bandes larges.

Des études plus précises ont été réalisées en ce qui concerne les précédents virus HIV-2 et SIV. La poursuite de l'étude des rétrovirus HIV-2 a également conduit à l'obtention de séquences d'ADN complémentaires (ADNc) des ARNs de leurs génomes. La séquence nucléotidique complète de l'ADNc d'un rétrovirus représentatif de la classe HIV-2 (HIV-2 ROD) a été déposée le 21/02/- 1986 à la CNCM sous le n° I-522, sous le nom de référence LAV-II ROD).

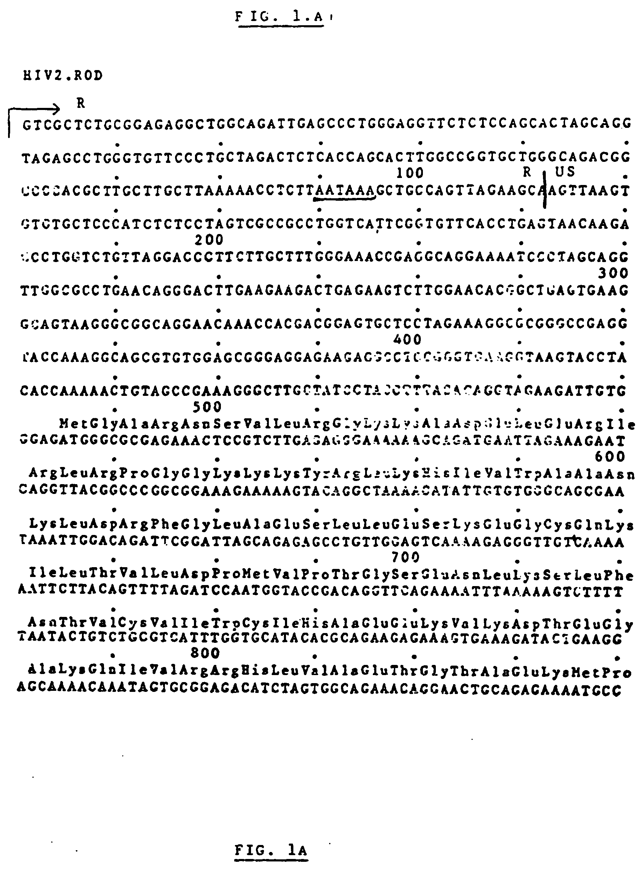

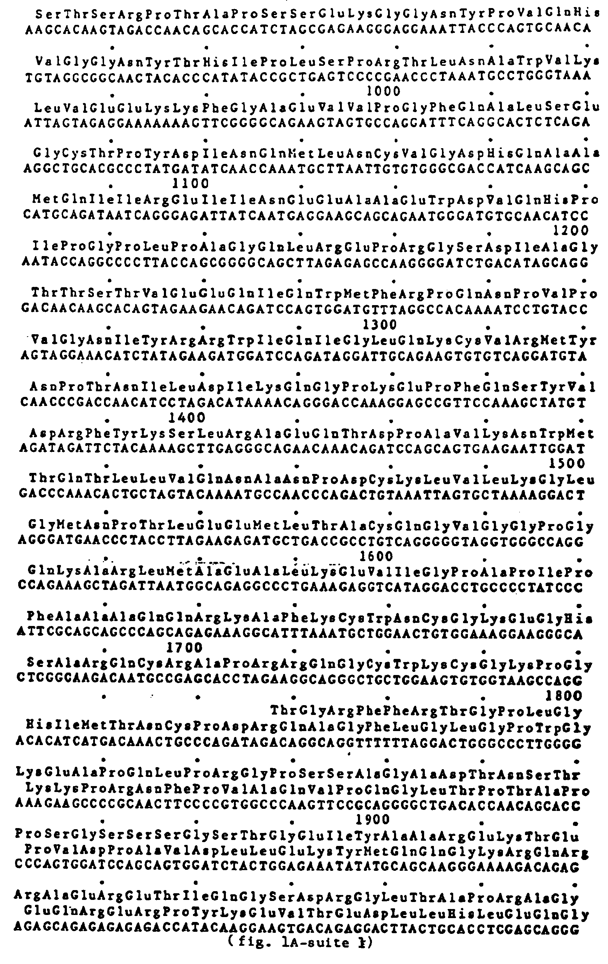

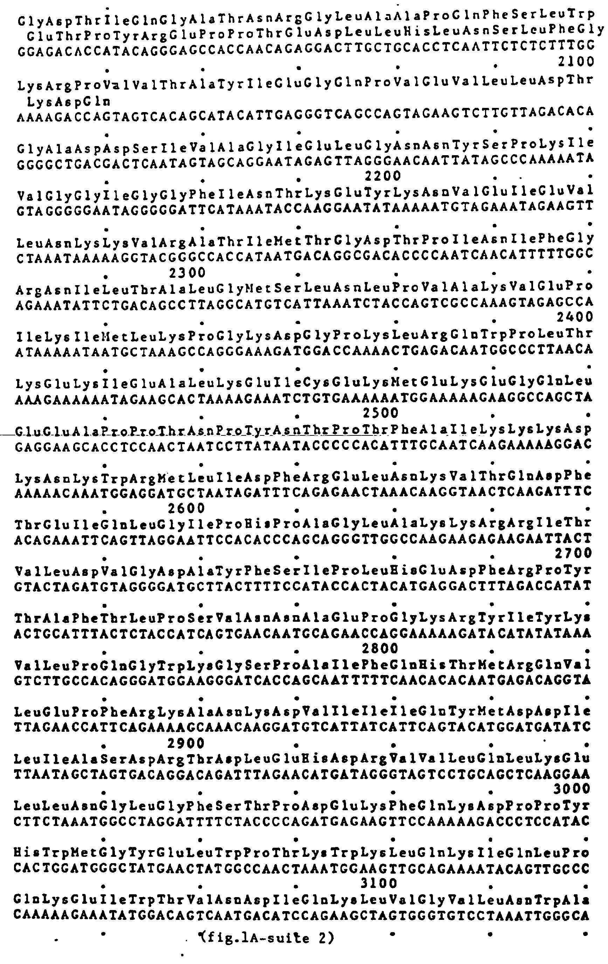

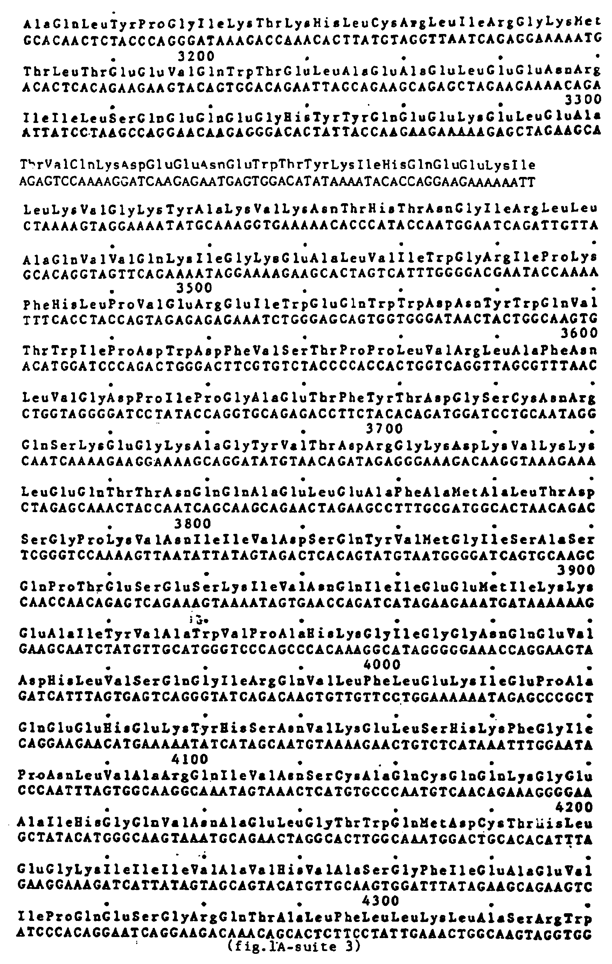

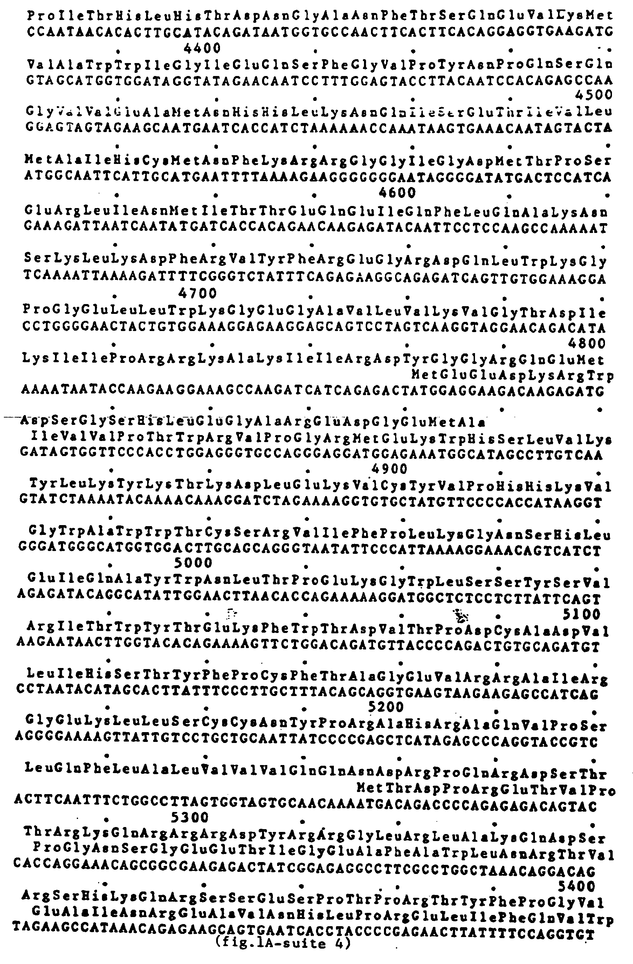

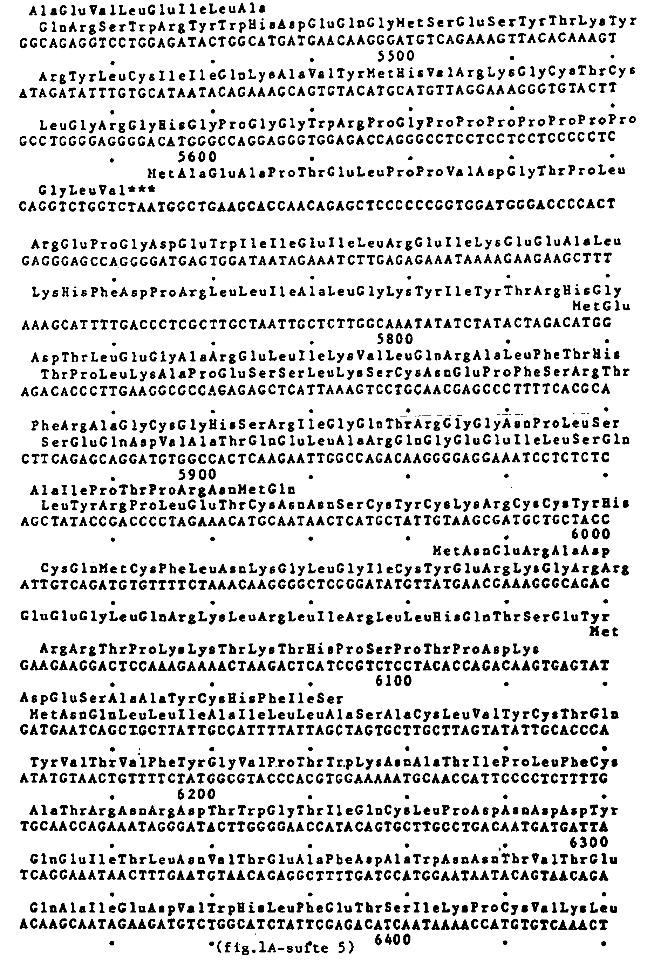

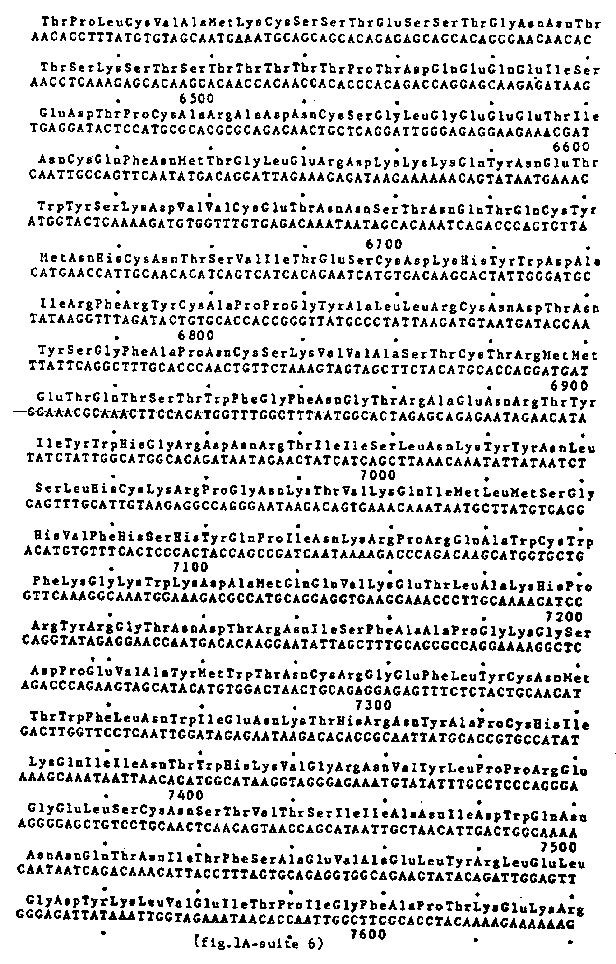

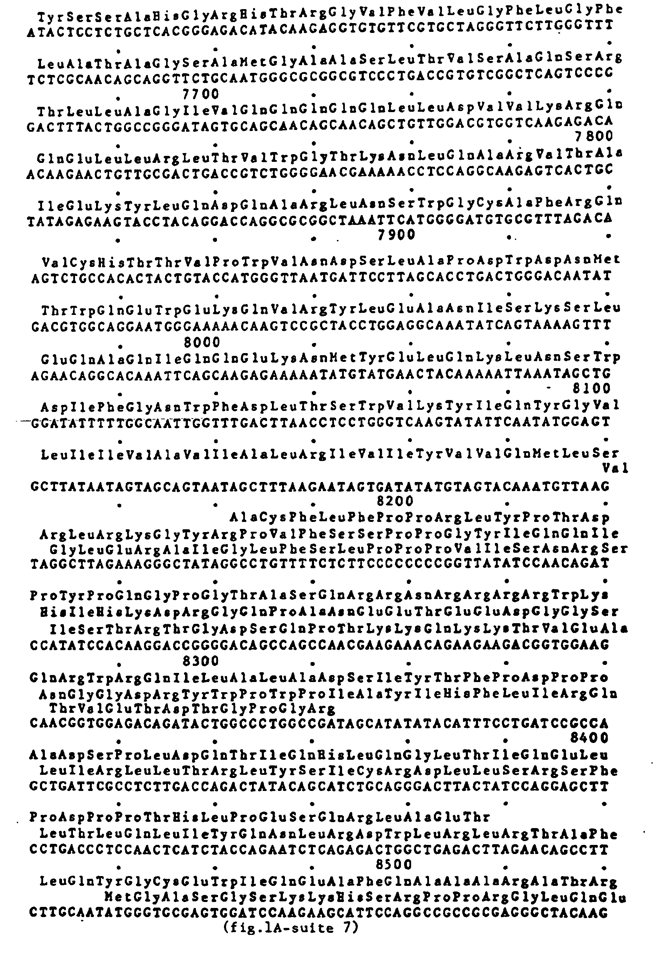

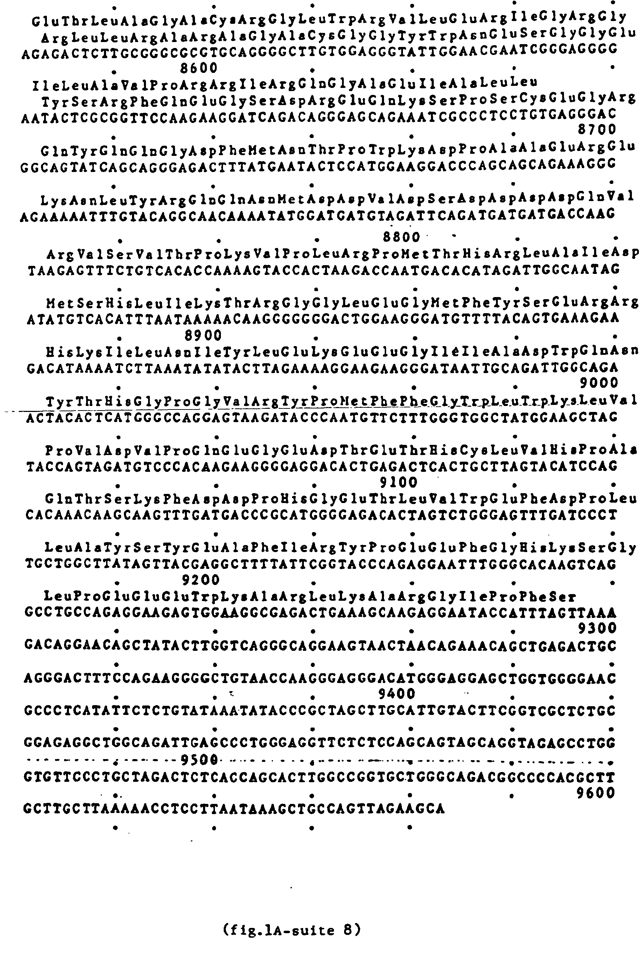

Cette séquence nucléotidique et les phases de lecture ouverte qu'elle contient sont indiqués à la figure 1A.

En outre, la poursuite de l'étude d'autres rétrovirus a également permis d'aboutir à l'aboutir à l'obtention de leurs séquences nucléotidiques complètes. Il en est en particulier ainsi de l'ADNc dérivé de l'ARN génomique de SIV.

Le clonage et le séquençage du virus SIV-1mac qui ont permis l'obtention de sa séquence nucléotidique ont été réalisés dans les conditions suivantes :
L'ADN de cellules HUT 78 infectées par le virus SIV (isolat STLV-III mac 142-83 décrit par Daniel et al.(1985) Science, 228, p.1201-1204, digéré partiellement par l'enzyme de restriction Sau3A a été cloné au site BamHI du bactériophage vecteur Lambda ELBL3 pour constituer une banque génomique. Les 2 millions de phages recombinants de la banque génomique ainsi constituée ont été criblés in situ en conditions de sécurité P3, à l'aide de séquences du virus HIV2 provenant des clones lambda-ROD4, lambda-ROD35 et E2 (Clavel et al. (1986-Nature, 324, p.691.) et nick-translatées.
L'hybridation a été réalisée en 5xSSC à 50°C et les lavages en 2xSSC à 50°C. Un seul clone contenant l'ensemble des séquences virales a été obtenu. Ce clone est désigné par lambda-SIV-1. L'insérat du phage lambda-SIV-1 mesure 16,5 kb au total et comprend un provirus intégré auquel manquent seulement les 250 premières bases du LTR gauche, alors que le LTR droit est complet.
Le provirus intégré a été séquencé par la méthode des didéoxynucléotides après sous-clonage de fragments aléatoires dans le phage M13mp8. 300 sous-clones ont été analysés.
Des fragments d'ADNc provenant du clone Lambda SIV-1 insérés dans des plasmides pSIV-1.1 et pSIV-1.2 ont été déposés à la CNCM le 15 Avril 1987, sous les numéros I-658 (pSIV-1.1) et I-659 (pSIV-1.2).

Les résultats ont été mentionnés dans les figures décrites ci-après.

La figure 1B représente la séquence nucléotidique du génome viral de SIV et les séquences qui en sont déduites pour les protéines virales correspondant aux produits des gènes gag, pol, env, Q, X, R, tat, art, F.

Les figures 3 à 11 et la figure 1C représentent les comparaisons des produits théoriques des gènes viraux et des LTR entre HIV2 et SIVmac. λSIV-1).

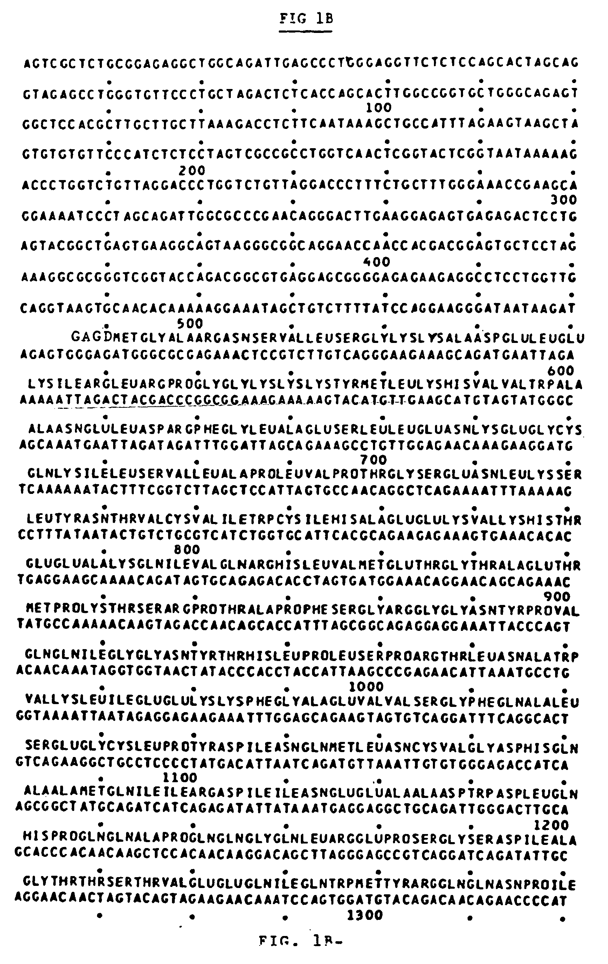

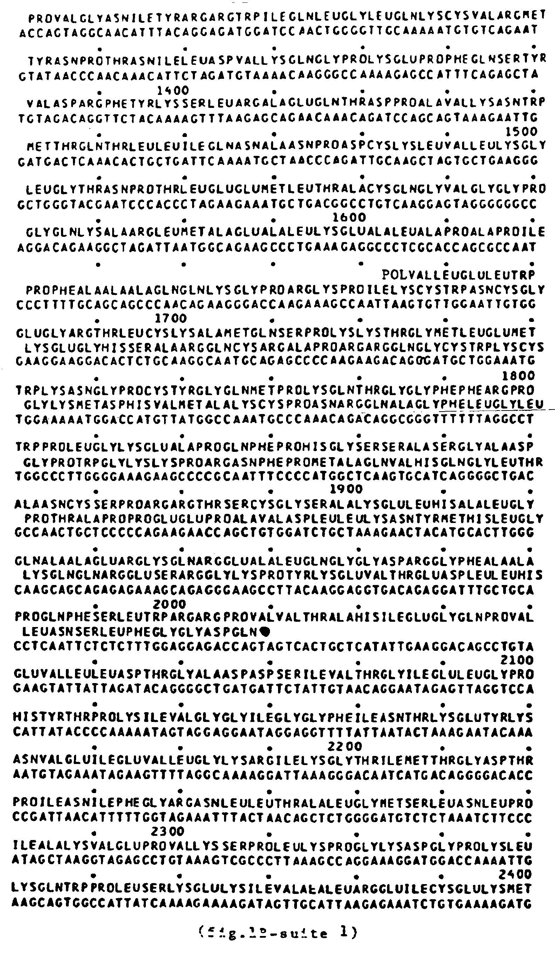

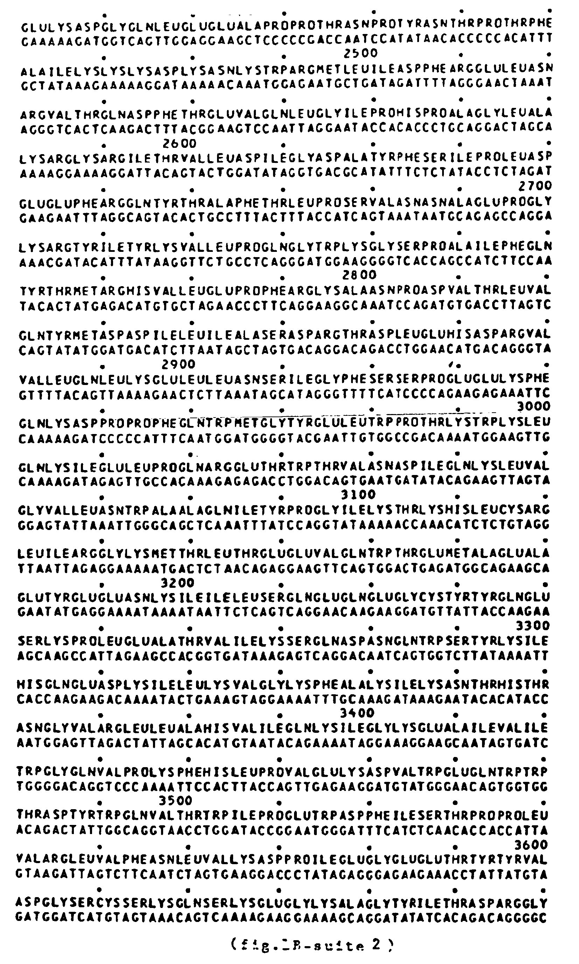

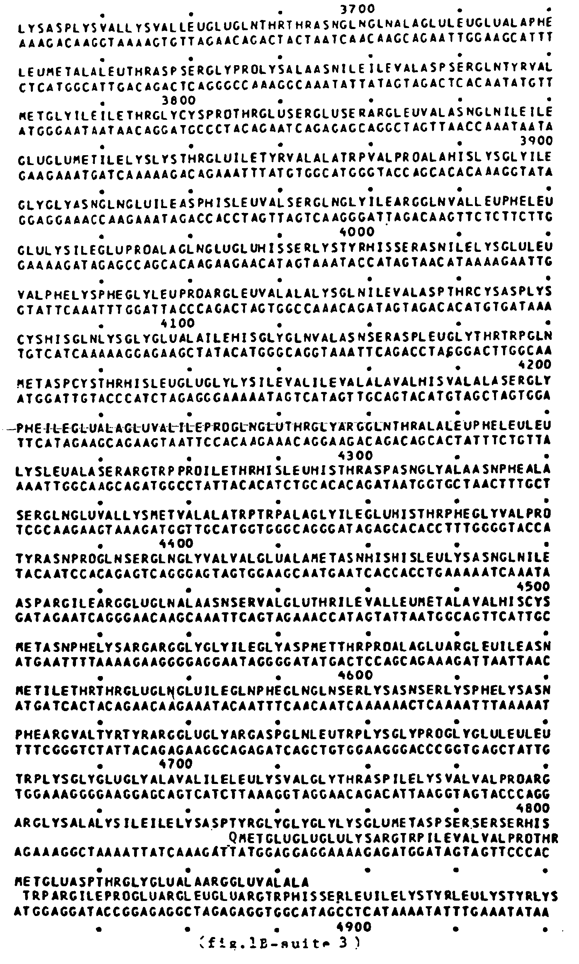

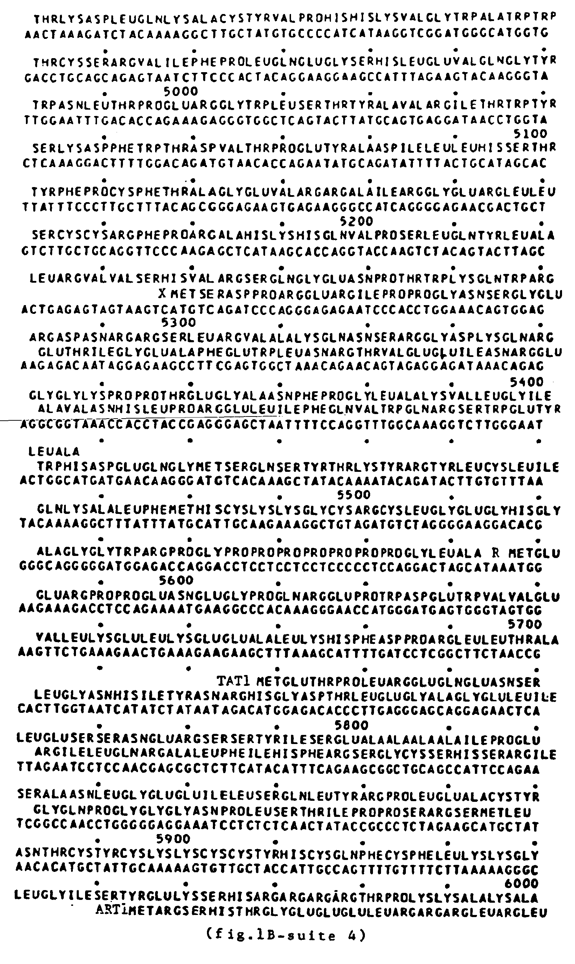

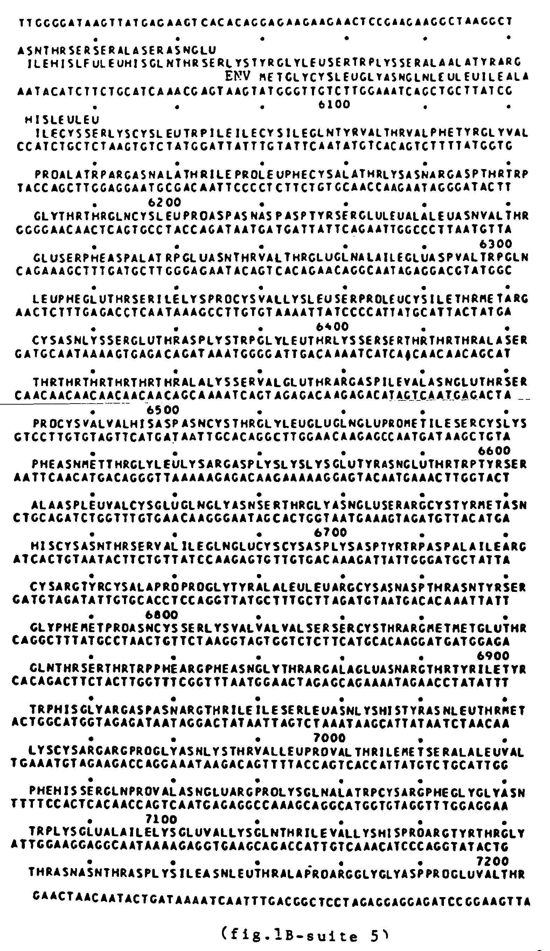

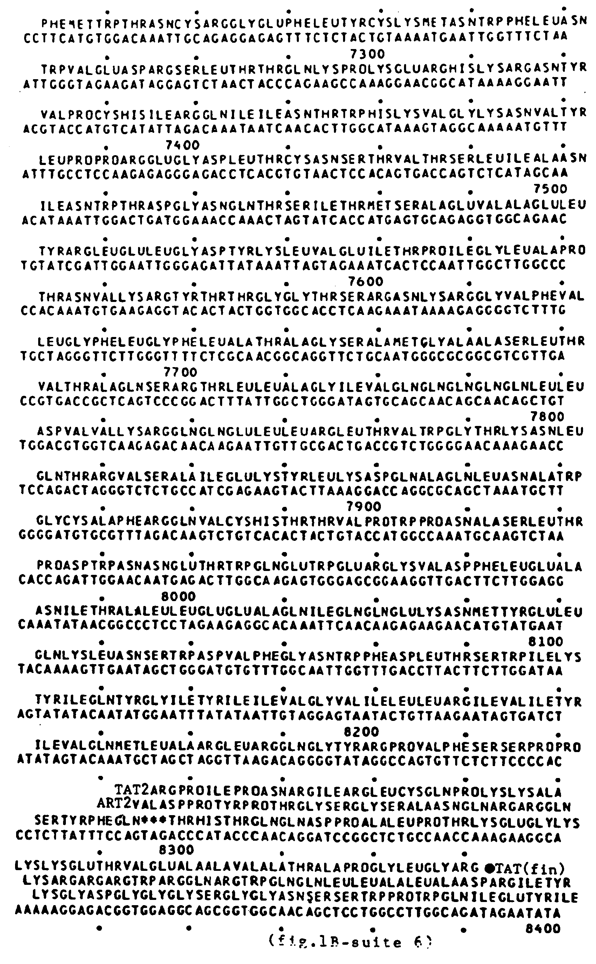

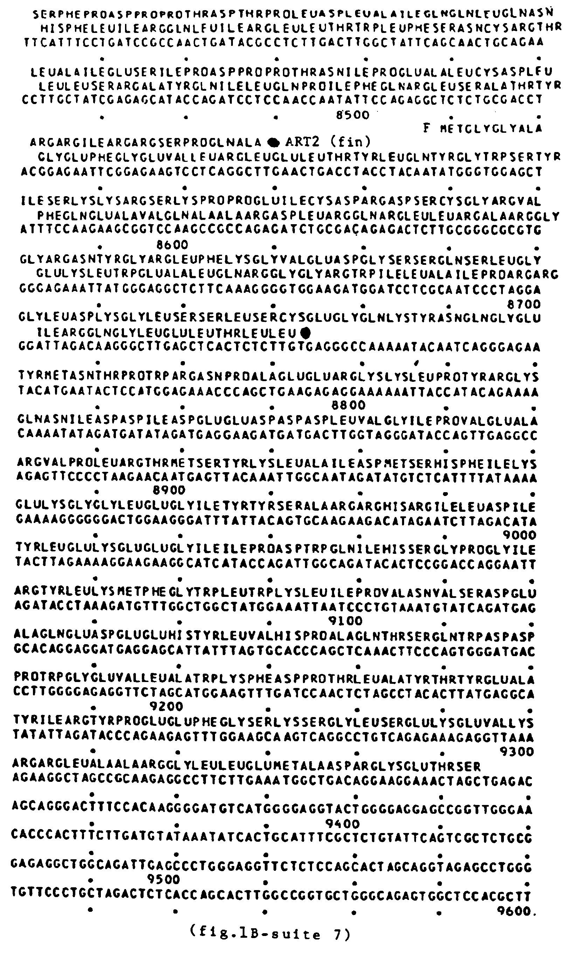

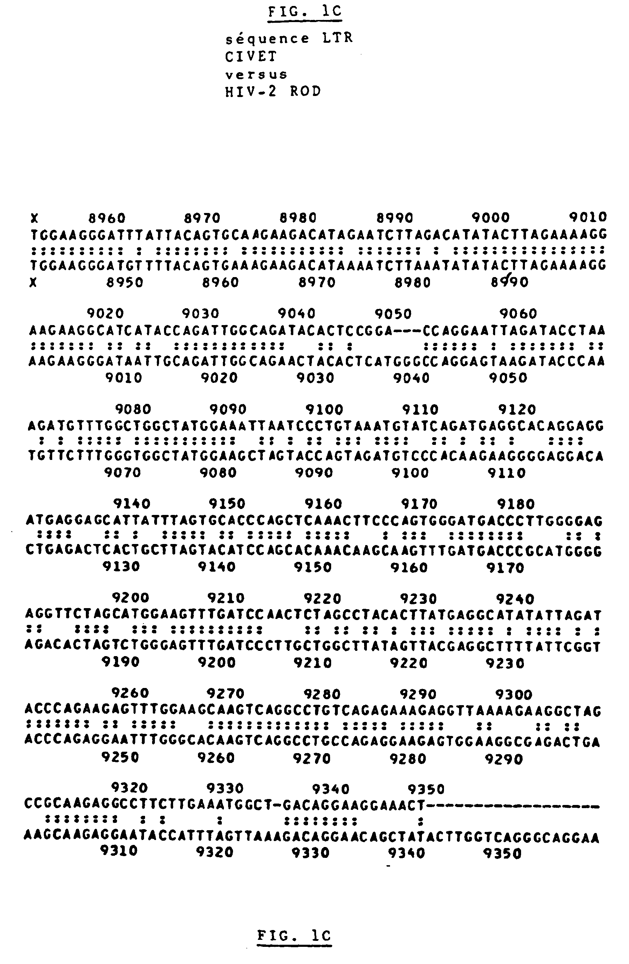

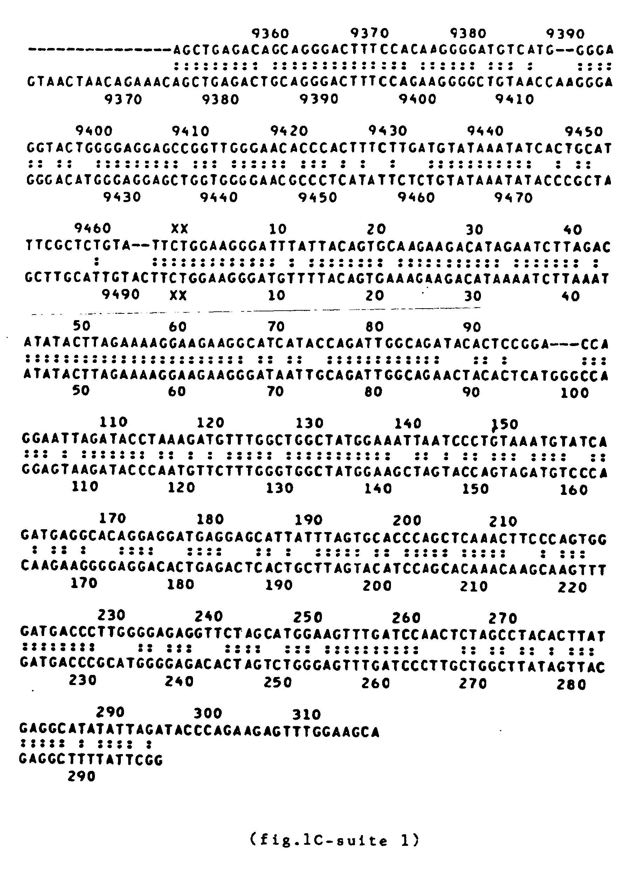

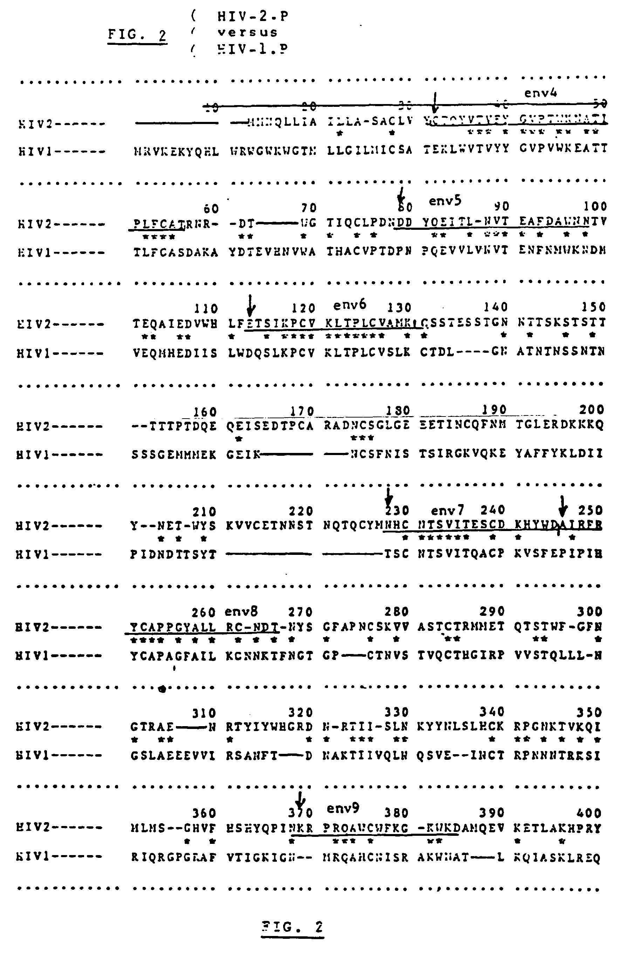

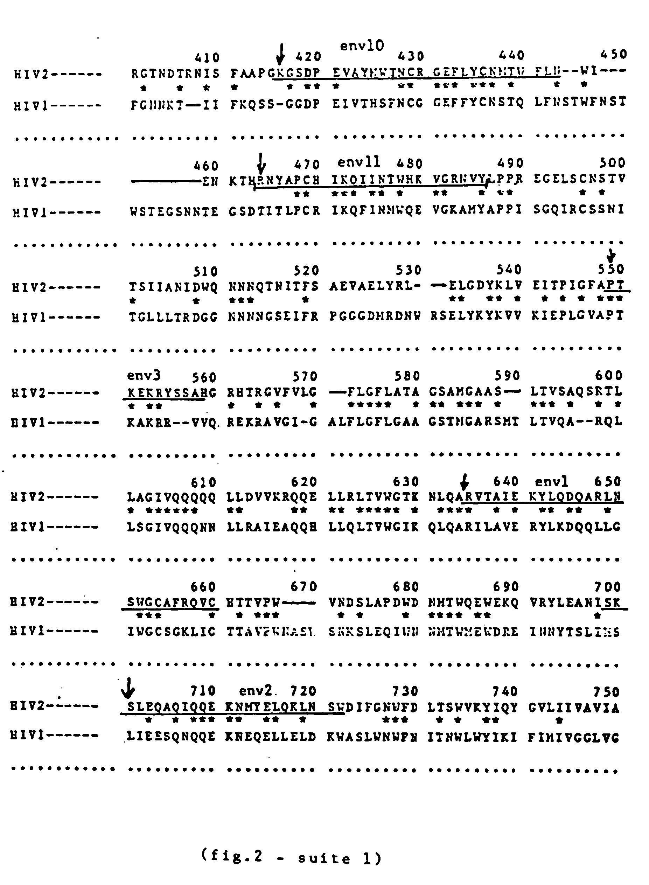

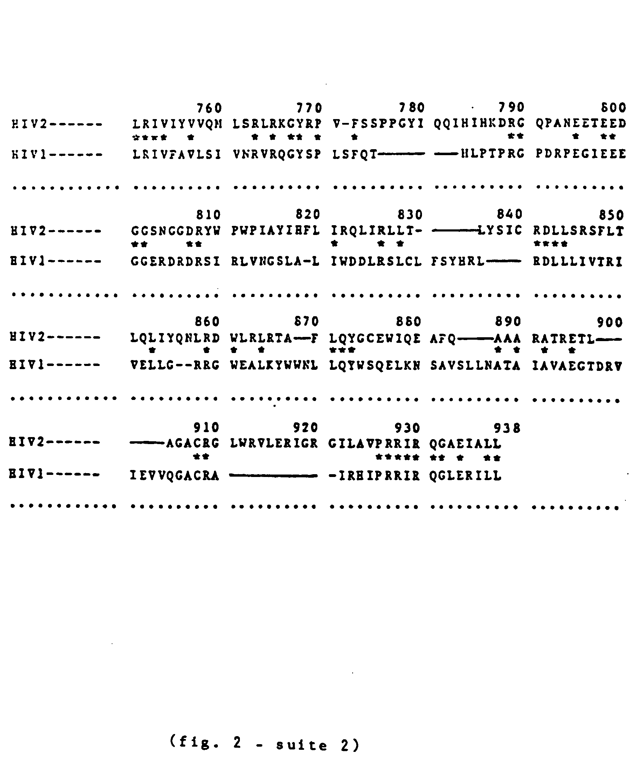

La demande décrit les fragments d'ADNc déduits de l'ADNc issu du génome entier de SIV-1, ces fragments contenant une ou plusieurs séquences issues de la séquence complète d'ADNc et qui codent pour des peptides intéressants de l'invention. Ces séquences sont indiquées à la figure 1B et, à la figure 1C pour ce qui a trait à la séquence LTR du virus,

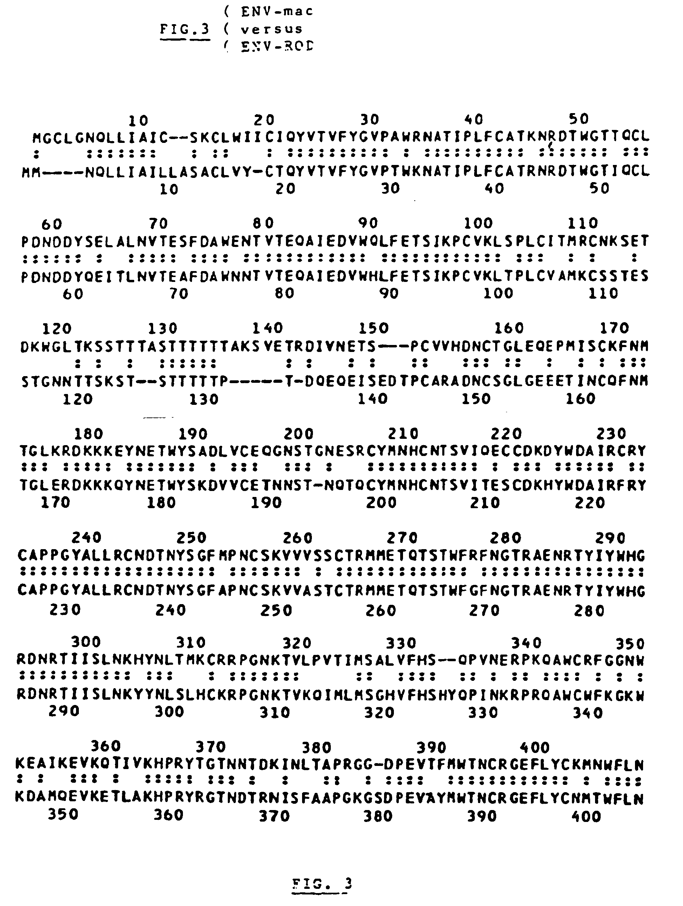

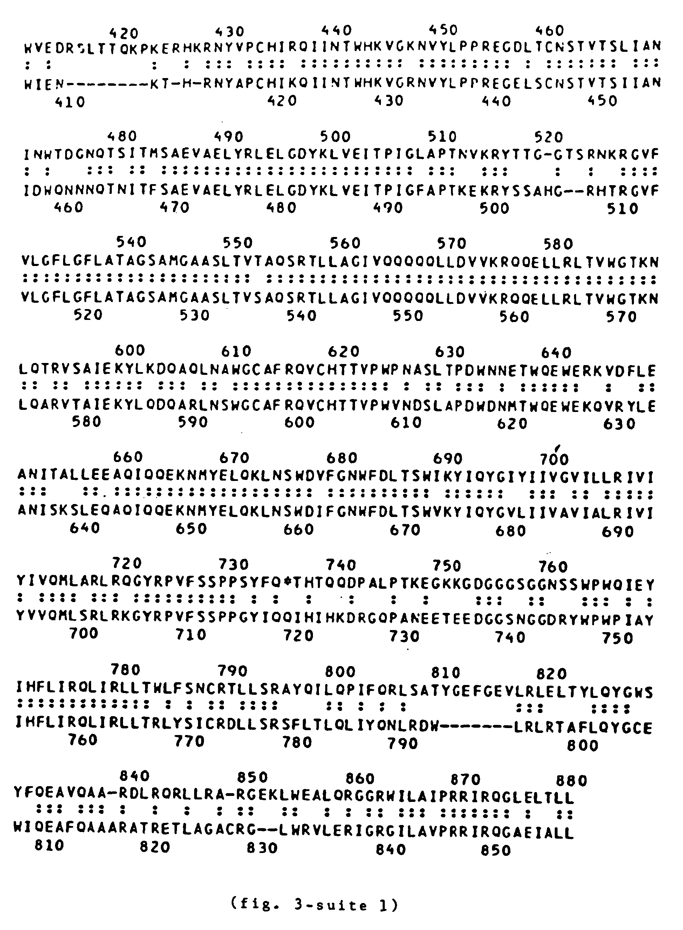

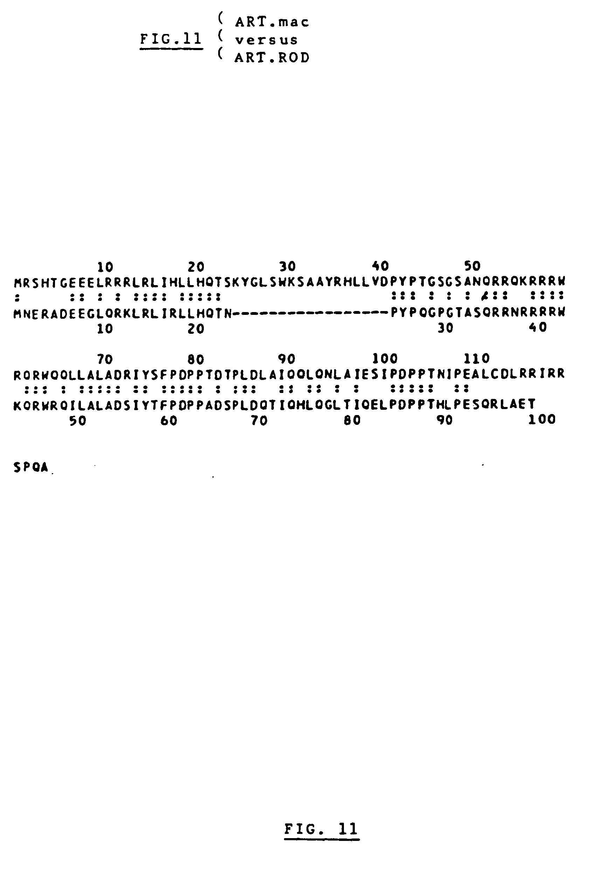

Les séquences nucléiques de l'ADNc de SIV ont été placées en correspondance avec les séquences nucléiques du virus HIV-2 ROD pour ce qui concerne la séquence LTR (figure 1C). Cette présentation que l'on retrouve pour le génome entier en rapprochant la figure 1B des figures 3 à 11 permet de repérer ou de déduire les acides nucléiques ayant des éléments de structure essentiels communs aux deux virus.

L'invention concerne naturellement aussi l'utilisation des cADNs issus de SIV ou de leurs fragments (ou de recombinants les contenant) en tant que sondes, pour le diagnostic de la présence ou non de virus HIV-2 dans des échantillons de sérums ou d'autres liquides ou tissus biologiques obtenus à partir de patients suspectés d'être porteurs du virus HIV-2. Ces sondes sont de préférence marquées également (marqueurs radio-actifs, enzymatiques, fluorescents, etc.). Des sondes particulièrement intéressantes pour la mise en oeuvre du procédé de diagnostic du virus HIV-2 ou d'un variant de HIV-2 peuvent être caractérisées en ce qu'elles comprennent la totalité ou une fraction de l'ADNc complémentaire du génome du virus SIV ou encore notamment les fragments recombinants contenus dans divers clones.

Les sondes mises en oeuvre dans ce procédé de diagnostic du virus HIV-2 et dans les kits de diagnostic ne sont en aucune façon réduites aux sondes décrites précédemment. Elles comprennent au contraire toutes les séquences nucléotidiques issues du génome du virus SIV, d'un variant de SIV ou d'un virus proche par sa structure, dès lors qu'elles permettent la détection dans des fluides biologiques de personnes susceptibles de développer un SIDA, d'anticorps dirigés contre un HIV-2 ou d'un virus qui en est proche.

La détection peut être réalisée de toutes façons en soi connues. Elle peut comprendre une mise en contact de ces sondes soit avec les acides nucléiques obtenus à partir des cellules contenues dans ces sérums ou autres milieux biologiques, par exemple liquides céphalo-rachidiens, salives, etc... Elle peut aussi comprendre une mise en contact de ces sondes avec ces milieux eux-mêmes dès lors que leurs acides nucléiques ont été rendus accessibles à l'hybridation avec ces sondes, et ce dans des conditions permettant l'hybridation entre ces sondes et ces acides nucléiques. L'étape finale du diagnostic in vitro comprend alors la détection de l'hybridation éventuellement produite. Le susdit diagnostic mettant en jeu des réactions d'hybridation peut également être réalisé à l'aide de mélanges de sondes respectivement originaires d'un HIV-2 et d'un SIV-1 ou d'un HIV-1, d'un HIV-2 et d'un SIV, dès lors qu'il n'est pas nécessaire de faire une différence entre le type de virus recherché.

D'une façon générale, le procédé de diagnostic de la présence ou non du virus HIV-2 ou d'un variant dans des échantillons de sérums ou d'autres liquides ou tissus obtenus à partir de patients suspectés d'être porteurs du virus HIV-2 comprend les étapes suivantes :
1/ au moins une étape d'hybridation conduite dans des conditions stringentes, par mise en contact de l'ADN de cellules de l'échantillon du patient suspect avec l'une des susdites sondes marquées sur une membrane approprié,
2/ le lavage de ladite membrane avec une solution assurant la conservation de ces conditions stringentes de l'hybridation,
3/ la détection de la présence ou non du virus HIV-2 par une méthode d'immunodétection.

Dans un autre mode de réalisation préféré du procédé selon l'invention l'hybridation précitée est conduite dans des conditions non stringentes et le lavage de la membrane est réalisé dans des conditions adaptées à celles de l'hybridation.

Les études comparatives qui ont aussi permis d'aboutir à des résultats relatifs aux protéines de noyau (core), ci-après dénommées "protéines gag" et aux protéines d'enveloppes, ci-après dénommées "protéines env", ont également été rapportés dans la demande de brevet européen n° 87/400.151.4, déjà citée. Ces résultats montrent que les protéines du noyau (protéines gag) dans HIV-2 présentent des différences moins accentuées par rapport à celles des virus HIV-1, que les protéines d'enveloppe (protéines env). Globalement les protéines env dans HIV-2 se sont révélées présenter des parentés immunologiques extrêmement faibles, sinon inexistentes, avec les protéines env correspondantes des virus HIV-1.

Au contraire des études comparatives effectuées entre les structures des séquences d'ADNc des virus HIV-2 et SIV permettent de mettre en évidence certaines caractéristiques communes qui apparaissent au niveau des protéines.

Globalement, les protéines de HIV-2 et de SIV-1 montrent des parentés immunologiques importantes.

La glycoprotéine majeure d'enveloppe de HIV-2 s'est révélée être plus proche immunologiquement de la glycoprotéine majeure d'enveloppe de SIV que de la glycoprotéine majeure d'enveloppe de HIV-1.

Ces constatations s'imposent non seulement au niveau des poids moléculaires : 130-140 kilodaltons pour les glycoprotéines majeures de HIV-2 et de SIV contre environ 110 pour la glycoprotéine majeure d'enveloppe de HIV-1, mais aussi au niveau des propriétés immunologiques, puisque des sérums prélevés à partir de malades infectés par HIV-2, et plus particulièrement des anticorps formés contre la gp140 de HIV-2 reconnaissent la gp140 de SIV-1mac, alors que dans des essais semblables les mêmes sérums et les mêmes anticorps de HIV-2 ne reconnaissent pas la gp110 de HIV-1. Mais les sérums anti-HIV-1 qui n'ont jamais réagi avec la gp140 de HIV-2 précipitent une protéine de 26 Kdal marquée par la ³⁵S-cystéine, contenue dans les extraits de HIV-2.

La protéine majeure du noyau (core) de HIV-2 semble présenter un poids moléculaire moyen (environ 26.000) intermédiaire entre celui de la p25 de HIV-1 et la p27 de SIV.

Ces observations résultent des essais réalisés avec des extraits viraux obtenus à partir du HIV-2 isolé à partir de l'un des patients susmentionnés. Des résultats similaires ont été obtenus avec des extraits viraux du HIV-2 isolé à partir du second patient.

Des études plus poussées ont conduit les inventeurs à reconnaître une première classe de peptides ayant des séquences d'aminoacides soit identiques, soit proches de séquences contenues à l'intérieur des structures des protéines gag et env de HIV-2 ou de SIV voire de HIV-1. Ces peptides sont notamment applicables au diagnostic d'une infection chez l'homme par le virus HIV-2 ou de l'un de ses variants. , propriées correspondantes.

L'invention concerne également les acides nucléiques contenant une ou plusieurs séquences issues de la séquence de l'ADNc du virus HIV-2 ROD. Ces séquences repérées par la numérotation figurant sur la séquence précédemment décrite, codent pour certains peptides intéressants de l'invention.

| | | | | |
|---|---|---|---|---|
| Séquence | codant pour | env1 | nucléotides | 7850 à 7927 |
| " | " | env2 | " | 8030 à 8095 |
| " | " | env3 | " | 7601 à 7636 |
| " | " | env4 | " | 6170 à 6247 |
| " | " | env5 | " | 6294 à 6349 |
| " | " | env6 | " | 6392 à 6445 |
| " | " | env7 | " | 6724 à 6763 |
| " | " | env8 | " | 6794 à 6838 |
| " | " | env9 | " | 7112 à 7162 |
| " | " | env10 | " | 7253 à 7336 |
| " | " | env11 | " | 7358 à 7426 |
| " | " | gag1 | " | 1535 à 1597 |

L'invention concerne enfin les acides nucléiques correspondants du virus SIV, contenant une ou plusieurs séquences issues de l'ADNc du virus SIV-1. Ces séquences codant pour les peptides env1 à env11 et gag1 peuvent être repérés sur la figure 3 par comparaison avec les séquences correspondantes décrites pour HIV-2.

Il va de soi que l'invention concerne les acides nucléiques correspondant à des séquences placées en des régions analogues des ADNc dérivés de variants de HIV-2 ROD ou de SIV, ainsi que tous les acides nucléiques dont les modifications vis à vis des précédents résulteraient de la mise à profit de la dégénérescence du code génétique.

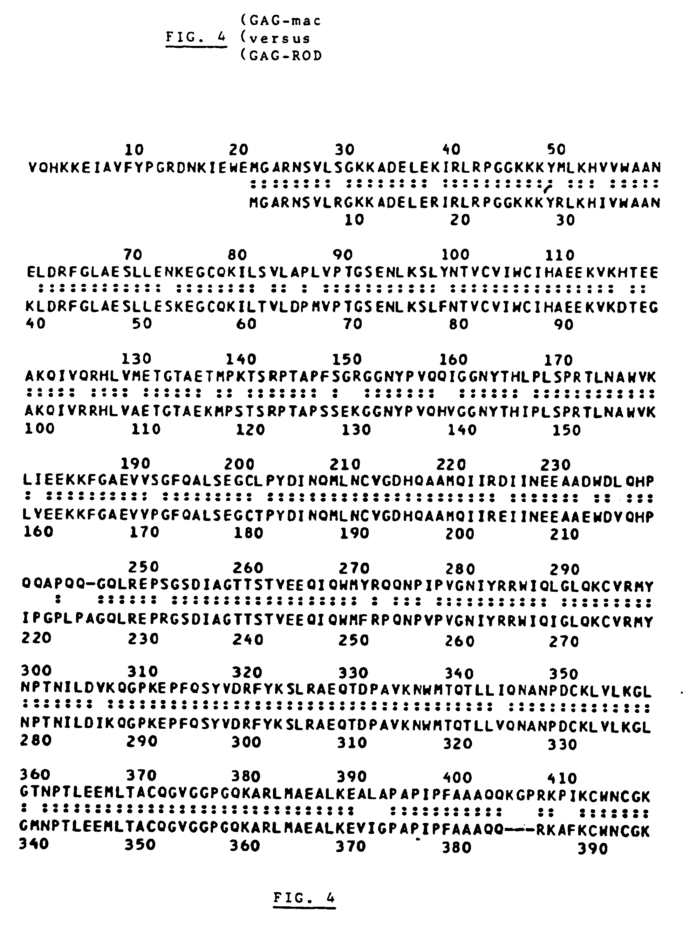

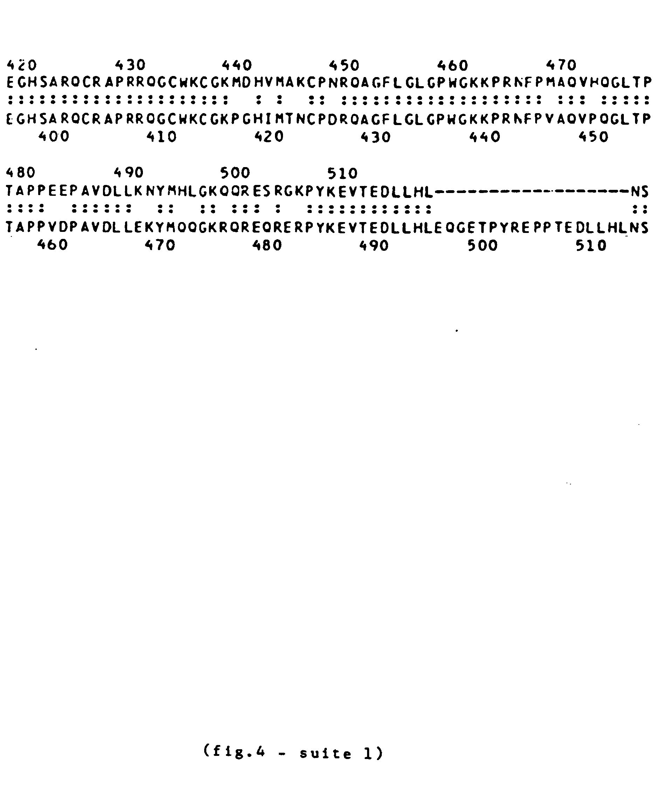

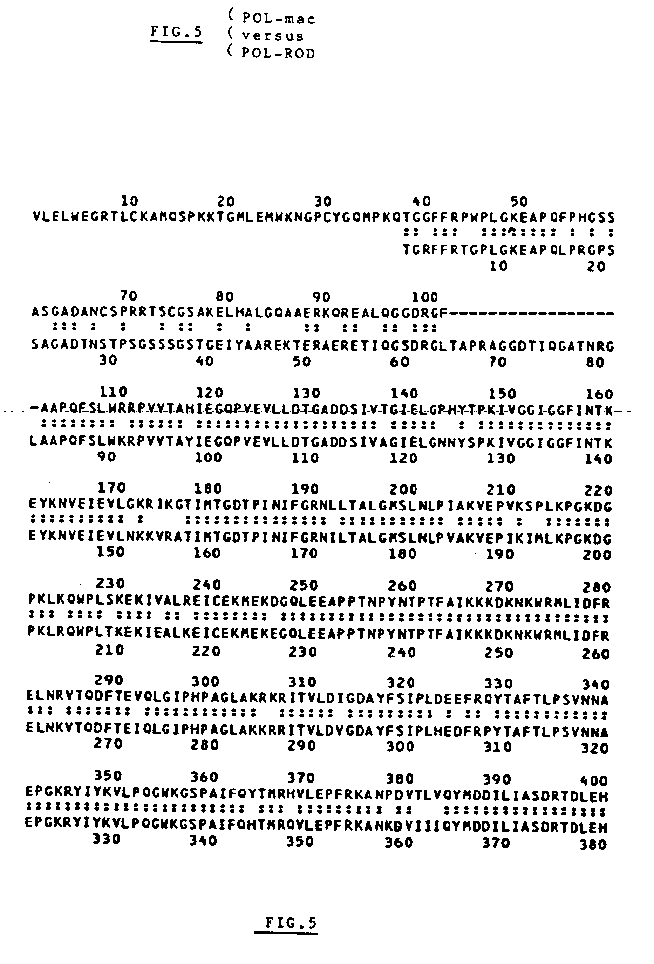

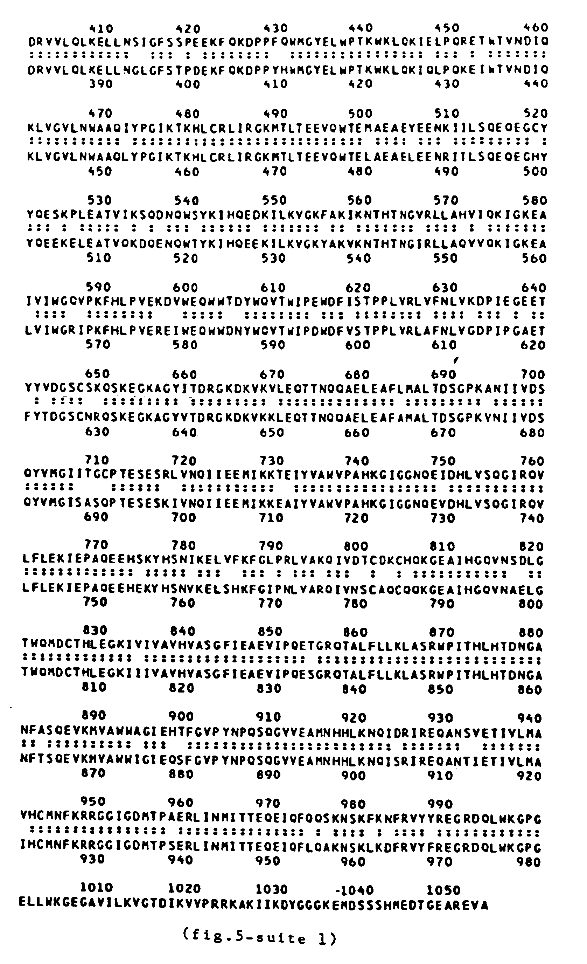

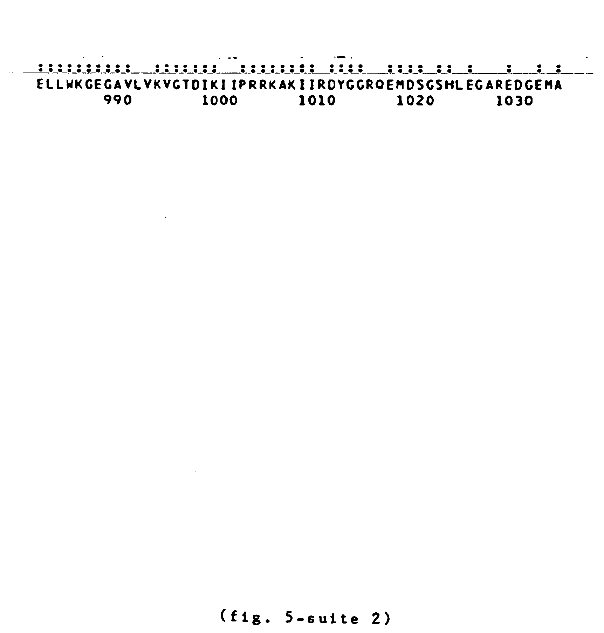

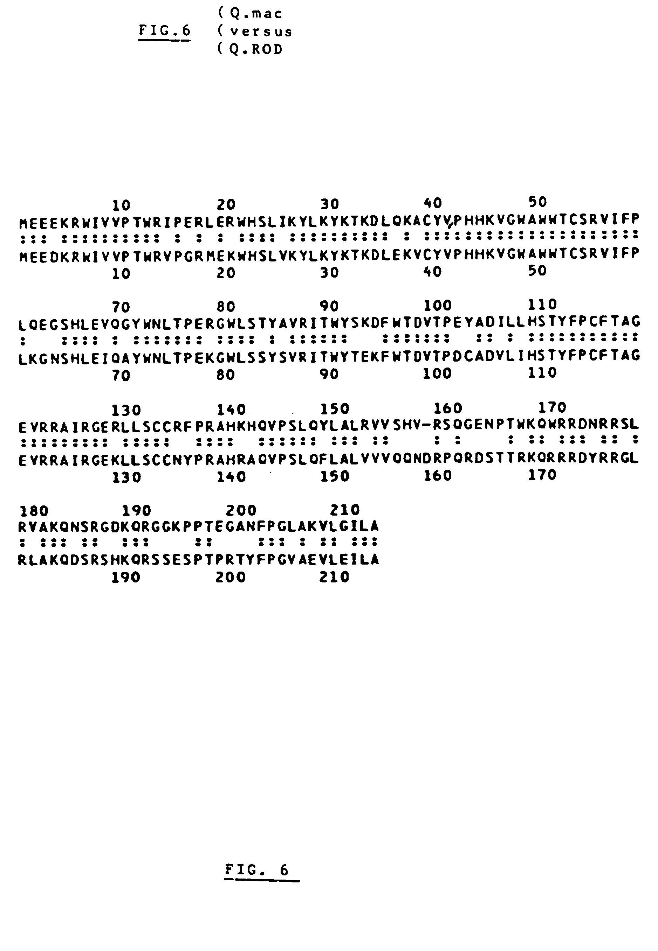

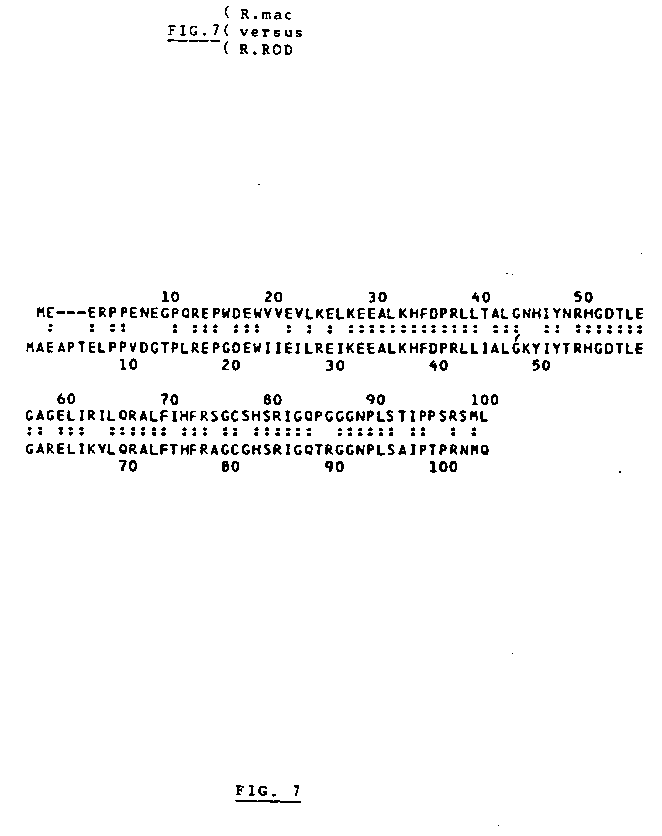

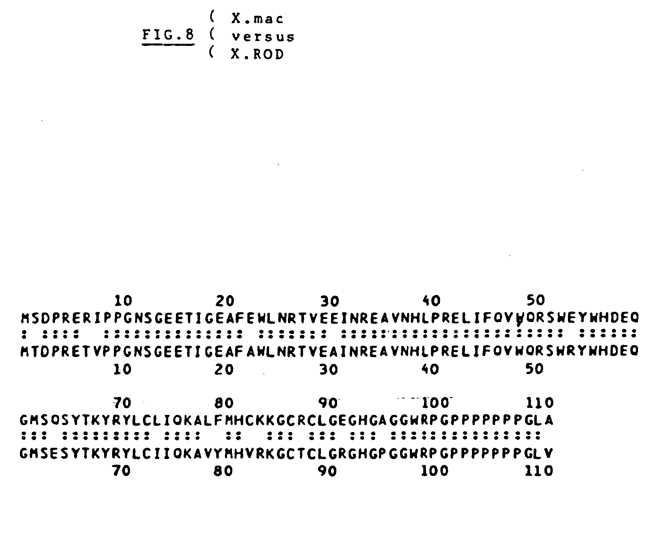

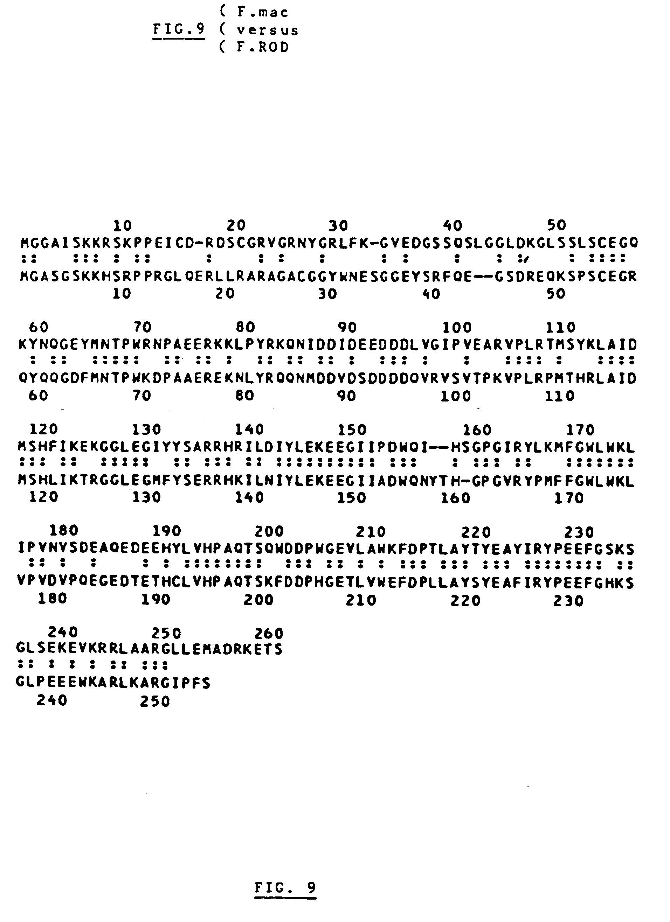

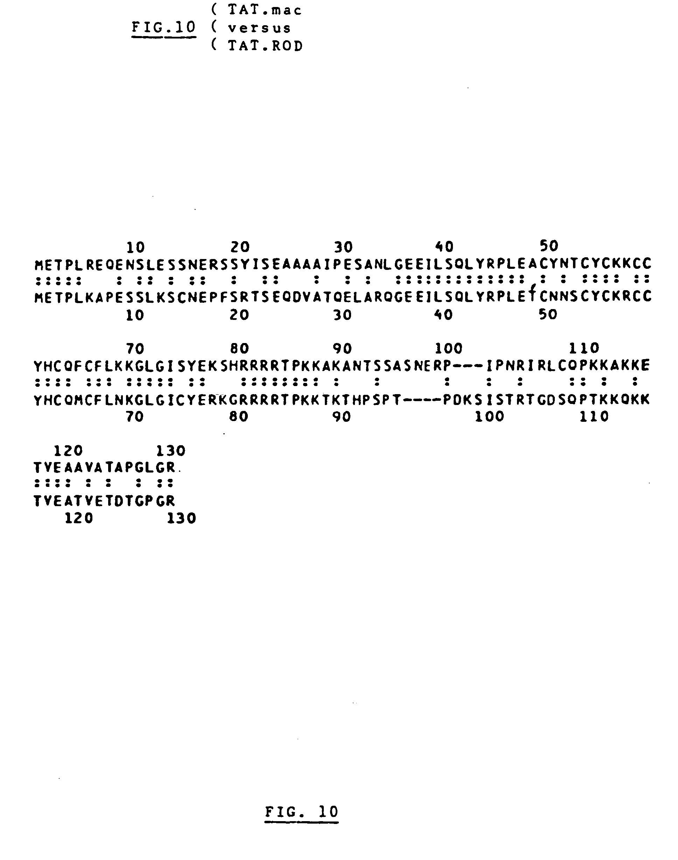

En particulier la demande décrit
1/ les protéines et glycoprotéines de l'enveloppe codées par le gène env et représentées à la figure 3,
2/ la protéine GAG représentée à la figure 4,
3/ la protéine POL représentée à la figure 5,
4/ la protéine Q représentée à la figure 6,
5/ la protéine R représentée à la figure 7,
6/ la protéine X représentée à la figure 8,
7/ la protéine F représentée à la figure 9,
8/ la protéine TAT représentée à la figure 10,

Les acides aminés des protéines précitées de SIV, ont été représentés en alignement avec les séquences d'acides aminés des protéines correspondantes du virus HIV-2 ; les points verticaux figurant entre les deux séquences correspondent aux acides aminés communs entre les protéines des deux virus.

Les séquences d'ADNc codant pour les protéines précitées apparaissent sur la figure 1B. L'invention concerne, outre les séquences nucléiques précitées toute séquence nucléiques modifiée, qui code également pour les protéines du rétrovirus SIV ou d'un variant.

Ces séquences d'ADNc repérées par la numérotation figurant sur les séquences décrites précédemment (figure 1B) sont les suivantes :
- séquence codant pour GAG, nucléotides 551 à 2068
- " " POL, " 1726 à 4893
- " " Q, " 4826 à 5467
- " " X, " 5298 à 5633
- " " R, " 5637 à 5939
- " " F, " 8569 à 9354
- " " TAT-1 " 5788 à 6084
- " " ART-1 " 6014 à 6130
- " " TAT-2 " 8296 à 8391
- " " ART-2 " 8294 à 8548
- " " ENV " 6090 à 8732

## Revendications

1. Séquence de nucléotides du rétrovirus SIV-1_{MAC}, **caractérisée en ce qu'**elle comprend l'une des séquences suivantes identifiées dans la figure 1B ou dans la figure 1C
| | |
|---|---|
| GAG s'étendant entre les nucléotides | 551 à 2068 |
| POL | 1726 à 4893 |
| Q | 4826 à 5467 |
| X | 5298 à 5633 |
| R | 5637 à 5939 |
| F | 8569 à 9354 |
| TAT-1 | 5788 à 6084 |
| ART-1 | 6014 à 6130 |
| TAT-2 | 8296 à 8391 |
| ART-2 | 8294 à 8548 |
| LTR | 8950 à 9468 et 1 à 316 |
| ENV | 6090 à 8732. |

2. Séquence de nucléotides selon la revendication 1, **caractérisée en ce qu'**elle comprend l'une des séquences suivantes identifiées dans la séquence de la figure 1B :
- séquence correspondant aux nucléotides 1538 à 1600
- séquence correspondant aux nucléotides 6144 à 6224
- séquence correspondant aux nucléotides 6270 à 6326
- séquence correspondant aux nucléotides 6363 à 6419
- séquence correspondant aux nucléotides 6720 à 6773
- séquence correspondant aux nucléotides 6774 à 6833
- séquence correspondant aux nucléotides 7101 à 7151
- séquence correspondant aux nucléotides 7239 à 7322
- séquence correspondant aux nucléotides 7374 à 7442
- séquence correspondant aux nucléotides 7572 à 7652.

3. Séquence de nucléotides **caractérisée en ce qu'**elle est constituée de l'une des séquences suivantes identifiées dans la séquence de la figure 1A :
- séquence correspondant aux nucléotides 7850 à 7927
- séquence correspondant aux nucléotides 8030 à 8095
- séquence correspondant aux nucléotides 7556 à 7636
- séquence correspondant aux nucléotides 6170 à 6247
- séquence correspondant aux nucléotides 6294 à 6349
- séquence correspondant aux nucléotides 6386 à 6442
- séquence correspondant aux nucléotides 6725 à 6778
- séquence correspondant aux nucléotides 6779 à 6838
- séquence correspondant aux nucléotides 7112 à 7162
- séquence correspondant aux nucléotides 7253 à 7336
- séquence correspondant aux nucléotides 7358 à 7426
- séquence correspondant aux nucléotides 1535 à 1597.

4. Séquence de nucléotides selon la revendication 1, **caractérisée en ce qu'**il s'agit de la séquence contenue dans le plasmide pSIV-1.1 (CNCM I-658) ou dans le plasmide pSIV-1.2 (CNCM I-659).

5. Séquence de nucléotides, **caractérisée en ce qu'**il s'agit de la séquence codante de l'un des polypeptides choisis parmi
POL_{MAC} représenté à la figure 5
Q_{MAC} représentée à la figure 6
R_{MAC} représentée à la figure 7
X_{MAC} représentée à la figure 8
F_{MAC} représentée à la figure 9
TAT_{MAC} représentée à la figure 10
ART_{MAC} représentée à la figure 11.

6. Séquence de nucléotides selon l'une quelconque des revendications 1 à 5, **caractérisée en ce qu'**elle est marquée.

7. Utilisation d'une séquence de nucléotides selon l'une quelconque des revendications 1 à 6 comme sonde pour la détection dans un échantillon biologique, d'une infection par un rétrovirus HIV-2.

8. Acide nucléique recombinant **caractérisé en ce qu'**il comprend une séquence de nucléotides selon l'une quelconque des revendications 1 à 5, insérée dans un acide nucléique provenant d'un vecteur.

9. Procédé de diagnostic de la présence ou non du virus HIV-2, dans des échantillons de sérums ou d'autres liquides ou tissus obtenus à partir de patients suspectés d'être porteurs du virus HIV-2 comprenant :
- au moins une étape d'hybridation conduite dans des conditions stringentes, par mise en contact de l'ADN de cellules de l'échantillon du patient suspect avec une sonde selon la revendication 6 sur une membrane appropriée,
- le lavage de ladite membrane avec une solution assurant la conservation de ces conditions stringentes de l'hybridation,
- la détection de la présence ou non du virus HIV-2 par une méthode d'immunodétection.

10. Procédé de diagnostic de la présence ou non du virus HIV-2, dans des échantillons de sérums ou d'autres liquides ou tissus obtenus à partir de patients suspectés d'être porteurs du virus HIV-2 comprenant :
- au moins une étape d'hybridation conduite dans des conditions non stringentes, par mise en contact de l'ADN de cellules de l'échantillon du patient suspecté d'être porteurs du virus HIV-2 avec une sonde selon la revendication 6 sur une membrane appropriée,
- le lavage de ladite membrane avec une solution assurant la conservation de ces conditions non stringentes de l'hybridation,
- la détection de la présence ou non du virus HIV-2 par une méthode d'immunodétection.

## Claims

1. A nucleotide sequence of the SIV-1_{MAC} retrovirus, **characterized in that** it comprises one of the following sequences identified in Figure 1B or in Figure 1C:
| | |
|---|---|
| GAG extending from nucleotides551 to 2068 | |
| POL | 1726 to 4893 |
| Q | 4826 to 5467 |
| X | 5298 to 5633 |
| R | 5637 to 5939 |
| F | 8569 to 9354 |
| TAT-1 | 5788 to 6084 |
| ART-1 | 6014 to 6130 |
| TAT-2 | 8296 to 8391 |
| ART-2 | 8294 to 8548 |
| LTR | 8950 to 9468 and |
| | 1 to 316 |
| ENV | 6090 to 8732. |

2. A nucleotide sequence according to claim 1, **characterized in that** it comprises one of the following sequences identified in the sequence of Figure 1B:
• the sequence corresponding to nucleotides 1538 to 1600;
• the sequence corresponding to nucleotides 6144 to 6224;
• the sequence corresponding to nucleotides 6270 to 6326;
• the sequence corresponding to nucleotides 6363 to 6419;
• the sequence corresponding to nucleotides 6720 to 6773;
• the sequence corresponding to nucleotides 6774 to 6833;
• the sequence corresponding to nucleotides 7101 to 7151;
• the sequence corresponding to nucleotides 7239 to 7322;
• the sequence corresponding to nucleotides 7374 to 7442;
• the sequence corresponding to nucleotides 7572 to 7652.

3. A nucleotide sequence, **characterized in that** it is constituted by one of the following sequences identified in the sequence of Figure 1A:
• the sequence corresponding to nucleotides 7850 to 7927;
• the sequence corresponding to nucleotides 8030 to 8095;
• the sequence corresponding to nucleotides 7556 to 7636;
• the sequence corresponding to nucleotides 6170 to 6247;
• the sequence corresponding to nucleotides 6294 to 6349;
• the sequence corresponding to nucleotides 6386 to 6442;
• the sequence corresponding to nucleotides 6725 to 6778;
• the sequence corresponding to nucleotides 6779 to 6838;
• the sequence corresponding to nucleotides 7112 to 7162;
• the sequence corresponding to nucleotides 7253 to 7336;
• the sequence corresponding to nucleotides 7358 to 7426;
• the sequence corresponding to nucleotides 1535 to 1597.

4. A nucleotide sequence according to claim 1, **characterized in that** it is the sequence contained in the plasmid pSIV-1.1 (CNCM I-658) or in the plasmid pSIV-1.2 (CNCM I-659).

5. A nucleotide sequence, **characterized in that** it is the sequence coding for one of the polypeptides selected from:
POL_{MAC} represented in Figure 5;
Q_{MAC} represented in Figure 6;
R_{MAC} represented in Figure 7;
X_{MAC} represented in Figure 8;
F_{MAC} represented in Figure 9;
TAT_{MAC} represented in Figure 10;
ART_{MAC} represented in Figure 11.

6. A nucleotide sequence according to any one of claims 1 to 5, **characterized in that** it is labelled.

7. Use of a nucleotide sequence according to any one of claims 1 to 6, as a probe for detecting an infection by an HIV-2 retrovirus in a biological sample.

8. A recombinant nucleic acid, **characterized in that** it comprises a nucleotide sequence according to any one of claims 1 to 5, inserted in a nucleic acid deriving from a vector.

9. A method for diagnosing the presence or otherwise of HIV-2 virus, in serum or other liquid or tissue samples obtained from patients suspected of being carriers of HIV-2 virus, comprising:
• at least one hybridization step carried out under stringent conditions, by bringing DNA from cells of a suspected patient's sample into contact with a probe in accordance with claim 6 on a suitable membrane;
• washing said membrane with a solution ensuring that said stringent hybridization conditions are conserved;
• detecting the presence or otherwise of HIV-2 virus using an immunodetection method.

10. A method for diagnosing the presence or otherwise of HIV-2 virus, in serum or other liquid or tissue samples obtained from patients suspected of being carriers of HIV-2 virus, comprising:
• at least one hybridization step carried out under non-stringent conditions, by bringing DNA from cells of a sample from a patient suspected of being a carrier of HIV-2 virus into contact with a probe in accordance with claim 6 on a suitable membrane;
• washing said membrane with a solution ensuring that said non-stringent hybridization conditions are conserved;
• detecting the presence or otherwise of HIV-2 virus using an immunodetection method.

## Patentansprüche

1. Nukleotidsequenz des Retrovirus SIV-1_{MAC}, **dadurch gekennzeichnet, dass** sie eine der folgenden Sequenzen umfasst, die in Figur 1B oder in Figur 1C dargestellt sind:
| | |
|---|---|
| GAG, die sich über die Nukleotide | 551 bis 2068 erstreckt, |
| POL | 1726 bis 4893 |
| Q | 4826 bis 5467 |
| X | 5298 bis 5633 |
| R | 5637 bis 5939 |
| F | 8569 bis 9354 - |
| TAT-1 | 5788 bis 6084 |
| ART-1 | 6014 bis 6130 |
| TAT-2 | 8296 bis 8391 |
| ART-2 | 8294 bis 8548 |
| LTR | 8950 bis 9468 und 1 bis 316 |
| ENV | 6090 bis 8732. |

2. Nukleotidsequenz gemäß Anspruch 1, **dadurch gekennzeichnet, dass** sie eine der folgenden Sequenzen umfasst, die in der Sequenz der Figur 1B dargestellt sind:
- Sequenz die den Nukleotiden 1538 bis 1600 entspricht,
- Sequenz die den Nukleotiden 6144 bis 6224 entspricht,
- Sequenz die den Nukleotiden 6270 bis 6326 entspricht,
- Sequenz die den Nukleotiden 6363 bis 6419 entspricht,
- Sequenz die den Nukleotiden 6720 bis 6773 entspricht,
- Sequenz die den Nukleotiden 6774 bis 6833 entspricht,
- Sequenz die den Nukleotiden 7101 bis 7151 entspricht,
- Sequenz die den Nukleotiden 7239 bis 7322 entspricht,
- Sequenz die den Nukleotiden 7374 bis 7442 entspricht,
- Sequenz die den Nukleotiden 7572 bis 7652 entspricht.

3. Nukleotidsequenz, **dadurch gekennzeichnet, dass** sie aus einer der folgenden Sequenzen besteht, die in der Sequenz der Figur 1A dargestellt sind:
- Sequenz, die den Nukleotiden 7850 bis 7927 entspricht,
- Sequenz, die den Nukleotiden 8030 bis 8095 entspricht,
- Sequenz die den Nukleotiden 7556 bis 7636 entspricht,
- Sequenz die den Nukleotiden 6170 bis 6247 entspricht,
- Sequenz die den Nukleotiden 6294 bis 6349 entspricht,
- Sequenz die den Nukleotiden 6386 bis 6442 entspricht,
- Sequenz die den Nukleotiden 6725 bis 6778 entspricht,
- Sequenz die den Nukleotiden 6779 bis 6838 entspricht,
- Sequenz die den Nukleotiden 7112 bis 7162 entspricht,
- Sequenz die den Nukleotiden 7253 bis 7336 entspricht,
- Sequenz die den Nukleotiden 7358 bis 7426 entspricht,
- Sequenz die den Nukleotiden 1535 bis 1597 entspricht.

4. Nukleotidsequenz gemäß Anspruch 1, **dadurch gekennzeichnet, dass** es sich dabei um die Sequenz handelt, die im Plasmid pSIV-1.1 (CNCM I-658) oder im Plasmid pSIV-1.2 (CNCM I-659) enthalten ist.

5. Nukleotidsequenz, **dadurch gekennzeichnet, dass** es sich um die codierende Sequenz eines Polypeptids handelt, das ausgewählt ist aus
POL_{MAC}, dargestellt in Figur 5,
Q_{MAC}, dargestellt in Figur 6,
R_{MAC}, dargestellt in Figur 7,
X_{MAC}, dargestellt in Figur 8,
F_{MAC}, dargestellt in Figur 9,
TAT_{MAC}, dargestellt in Figur 10,
ART_{MAC}, dargestellt in Figur 11.

6. Nukleotidsequenz gemäß einem der Ansprüche 1 bis 5, **dadurch gekennzeichnet, dass** sie markiert ist.

7. Verwendung einer Nukleotidsequenz gemäß einem der Ansprüche 1 bis 6 als Sonde zum Nachweis einer Infektion durch einen Retrovirus HIV-2 in einer biologischen Probe.

8. Rekombinante Nukleinsäure, **dadurch gekennzeichnet, dass** sie eine Nukleotid- sequenz gemäß einem der Ansprüche 1 bis 5 umfasst, die in eine Nukleinsäure eingefügt ist, die von einem Vektor stammt.

9. Diagnoseverfahren, ob der Virus HIV-2 in Proben von Serum oder anderen Flüssigkeiten oder Geweben vorliegt oder nicht, die von Patienten erhalten wurden, die im Verdacht stehen, Träger des Virus HIV-2 zu sein, umfassend:
- mindestens einen Hybridisierungsschritt, der unter stringenten Bedingungen durchgeführt wird, durch Inkontaktbringen von DNA aus Zellen der Probe des in Verdacht stehenden Patienten mit einer Sonde gemäß Anspruch 6 auf einer geeigneten Membran,
- Waschen dieser Membran mit einer Lösung, die die Aufrechterhaltung dieser stringenten Hybridisierungsbedingungen sicherstellt,
- Nachweis, ob der Virus HIV-2 vorliegt oder nicht, durch ein Immunnachweisverfahren.

10. Diagnoseverfahren, ob der Virus HIV-2 in Proben von Serum oder anderen Flüssigkeiten oder Geweben vorliegt oder nicht, die von Patienten erhalten wurden, die im Verdacht stehen, Träger des Virus HIV-2 zu sein, umfassend:
- mindestens einen Hybridisierungsschritt, der unter nicht-stringenten Bedingungen durchgeführt wird, durch Inkontaktbringen der DNA von Zellen der Probe des Patienten, der im Verdacht steht, Träger des Virus HIV-2 zu sein, mit einer Sonde gemäß Anspruch 6 auf einer geeigneten Membran,
- Waschen dieser Membran mit einer Lösung, die die Aufrechterhaltung dieser nicht-stringenten Hybridisierungsbedingungen sicherstellt,
- Nachweis, ob der Virus HIV-2 vorliegt oder nicht, durch ein Immunnachweisverfahren.
